# EUROPEAN PATENT SPECIFICATION

(11) **EP 0 487 590 B1**
(45) Date of publication and mention of the grant of the patent: **15.11.1995**
(21) Application number: 90912738.3
(22) Date of filing: 16.08.1990
(51) Int. Cl.: A61B 17/32

(54) **CATHETER ATHEROTOME**
KATHETER ZUR ATHEROTOMIE
ATHEROTOME A CATHETER

(30) Priority: 18.08.1989 US 395500
(43) Date of publication of application: 03.06.1992
(73) Proprietor: EVI CORPORATION, Portland OR 97219 (US)
(72) Inventor: REGER, Vincent, A., Portland, OR 97225 (US); KELLY, Thomas, L., West Linn, OR 97068 (US)
(74) Representative: Meddle, Alan Leonard
(86) International application number: PCT/US90/04638
(87) International publication number: WO 91/02494

(56) References cited:
- EP-A- 0 117 519
- US-A- 3 320 957
- US-A- 3 320 957
- US-A- 4 030 503
- US-A- 4 650 466
- US-A- 4 653 496
- US-A- 4 765 332
- US-A- 4 921 484

## Description

### Technical Field

The present invention relates to surgical apparatus and procedures, and particularly to a device for excising portions of the atherosclerotic plaque material causing stenosis in an artery.

### Background Art

Atherosclerosis (Greek for soft and hard deposits) is a condition which progressively affects many arteries of the body with advancing age. It ultimately produces thickening of the medial layer of the arterial wall, which may involve some or all of the circumference of the blood vessel. Eventually, narrowed internal diameter, or stenosis, of the artery results and restricts the flow of blood to the tissue beyond the stenosis, producing symptoms including angina or myocardial infarction in the heart, claudication or gangrene in the legs, etc., high blood pressure, or deterioration of kidney function.

The art and science involved in modern vascular surgery are comparatively young and began with the successful end-to-end repair of severed arteries in Korean war casualties. Atherosclerotic narrowing of arteries then could only be corrected by complete endarterectomy, which required a longitudinal incision through the entire narrowed segment of an artery. Exposure of an artery for this purpose was difficult, and the wounds resulting from the surgery were large. Although results were often gratifying, the practice was not widespread because of resulting problems such as pseudoaneurysms developing in endarterized segments and the potential for vessel wall dissection at the distal endpoint, and because of the difficulty posed both for the patient and for the surgeon. During the 1950's a variety of synthetic tubular grafts were introduced and perfected for partial arterial replacement and bypasses around stenoses. Because of the relative ease of such procedures by comparison with endarterectomy, bypass grafting soon became the dominant means of correcting arterial narrowing within the pelvis and thigh. Advances in surgical technique in the late 1960's made possible the use of the patient's own reversed saphenous vein to bypass occluded arteries in the heart and below the knee.

As the population of the United States has aged as a group, the manifestations of atherosclerosis have, as a group, become this nation's number one health problem in terms of both suffering and cost. While surgical bypass procedures using saphenous vein or prosthetic conduit remain the procedure of choice in most instances, newer technologies have evolved in the last decade to simplify the treatment of atherosclerotic stenoses in an attempt to reduce patient risk, reduce cost, and to make treatment available to more people. In carefully selected cases involving narrowing of short segments of the coronary, renal, iliac, and femoral arteries balloon dilation has been employed with some success. Generally, however, the duration of arterial patency resulting from such procedures is less than for bypass graft procedures. Utilization of lasers to open narrowed arteries has not yet proven to be clinically successful and is very expensive in all aspects.

In recent years a variety of atherectomy devices have been used experimentally in attempts to extend patency. Some of these devices include rotary cutting mechanisms, which restrict their use to stenoses of short length. Some are driven by high-speed electric motors which add to their complexity and increase the likelihood of breakdown while also reducing the amount of responsiveness and taking the ability to control the operation out of the surgeon's hands.

Manually-operated devices for relieving arterial stenoses are disclosed, for example, in Lary US-A-4,273,128, which discloses a device having a plurality of curved knife blades whose edges are directed radially outward, and Fischell et al. US-A-4,765,332, which discloses a catheter including a proximally-exposed annular cutting edge which is no greater in diameter than an outer sleeve of the catheter to which it is attached. Luther US-A-4,650,446, discloses a catheter which includes an expansible woven tube portion which can be used to abrade atherosclerotic plaque from the interior wall of the artery. Clark, III, US-A-4,020,847, discloses a catheter device including a slot having sharp edges extending longitudinally of the catheter to cut free dangling matter which might otherwise obstruct the lumen of an artery. Hoffman US-A-2,730,101 and 2,816,552 disclose teat bistoury devices including blades which can be bowed outwardly along the length of each blade to protrude radially. The devices are intended to be rotated to cut away restrictions in a milk canal of a cow's teat. Several prior art devices useful for manually opening venous valves are disclosed in Chin et al. US-A-4,739,760 and 4,768,508 and Reed US-A-4,655,217.

Chin US-A-4,559,927 discloses an endarterectomy apparatus including a center-pull annular cutter for removing arteriosclerotic material.

Rotary, mechanically operated devices are disclosed in such patents as Sokolik US-A-3,320,957, which discloses a device including an array of helical stationary blades inside which an oppositely-twisted helical rotor operates to shear material protruding inwardly between the stationary blades. Means are provided for axially compressing the device in order to flex the blade into a radially bowed arcuate configuration. Auth US-A-4,445,509 discloses a fluted rotary burr. Kensey US-A-4,589,412 and 4,631,052 disclose turbine-driven rotary devices for opening obstructed arteries, and Kensey et al. US-A-4,581,106 discloses another turbine-driven rotary cutting device.

Several devices for use in retrieving stones from within bodily passageways by entrapping the stones within baskets including arrays of helical wires are disclosed in Grayhack et al. US-A-4,611,594, Duthoy US-A-4,625,726, Dormia US-A-4,347,846 and US-A-4,612,931. Related devices are disclosed by McGirr US-A-4,807,626, and Hawkins, Jr. et al. US-A-4,790,812, which discloses a parachute-like basket carried on a distal end of a rotatable interior member of a catheter so that the parachute-like basket can retrieve particles cut free by the interior member of the catheter. Park US-A-3,704,711 discloses a device in which a radially outwardly disposed edge can be controllably concealed within a distal end of a catheter or exposed so that the blade can be used.

Balloon-tipped catheters are disclosed in Fogarty US-A-3,435,826, while Fogarty US-A-3,472,230 discloses a catheter including an umbrella-like skirt useful for retrieval of stones.

There still remains a need, however, for an improved atherectomy device which is simple in concept and operation, manually operable, and immediately responsive, and which is useful for all stenoses regardless of the length of the area of stenosis.

### Disclosure of the Invention

The present invention overcomes some of the shortcomings and disadvantages of the devices disclosed in the prior art by providing a catheter atherotome which is manually operable and by which a surgeon can plane away atherosclerotic plaque from within an artery by entering the artery with a catheter at a single point proximal to the plaque deposit. The plaque is cut away piecemeal, by serial pullback strokes of a basket knife carried on the distal end of the catheter and which is collapsible to a small diameter conforming to the diameter of the catheter itself. The basket knife of the catheter atherotome includes several blades which are aligned generally parallel with one another and in a generally helical arrangement about an inner portion of the catheter which extends distally beyond the distal end of an outer sheath portion of the catheter when the basket knife is in a radially contracted, relaxed configuration. Respective ends of each blade are attached to the inner member and the outer sheath of the catheter so that when the distal end of the inner member is moved closer to the distal end of the outer sheath the blade members are forced to bow outward, expanding the basket knife radially. When the blades are bowed outward, sharpened edges of the blades are exposed and directed in a direction more proximal than distal with respect to the catheter, so that moving the catheter proximally brings the edges to bear against atherosclerotic plaque deposits to cut them away from the interior of an artery, while moving them distally will not result in cutting any normal arterial intima.

In a preferred embodiment of the invention, both the longitudinal position and the rotational position of the inner member of the catheter are adjustable relative to the outer sheath, and the several blades are flexible, so that both the pitch and the amount of radial bowing of the blades are controllable. Preferably, the angle of attack of the sharpened edge is such that it will engage atherosclerotic plaque but not normal arterial lining tissue.

In one embodiment of the invention a tensioning bar is provided in a position ahead of the sharp edge of a blade to provide some tension in the tissue to be cut and thus achieve a cleaner, less traumatic cut.

Another embodiment of the invention utilizes a more rigid basket knife in which sharpened portions of the blades overlap one another to provide complete circumferential coverage of the interior of an artery.

Yet another embodiment of the basket knife portion of the device includes cutting edges over only about half of the basket circumference, so that atherectomy can be performed on one-half of the arterial wall in locations where atherosclerosis normally involves only the posterior one-half of the artery.

Preferably, a lumen is provided in the inner member of the catheter, and a balloon-tipped catheter such as a Fogarty® catheter or a guide wire-mounted endovascular filter may be utilized to collect shavings of plaque cut free by the catheter atherotome of the invention, so that the shavings can be surgically removed through an incision in the arterial wall after atherectomy has been performed.

In another embodiment of the invention a latex skirt is provided to surround the basket knife assembly except where the cutting edges are provided, so that the shavings are trapped within the basket during each cutting pass of the basket over the plaque. This embodiment is intended for use in smaller arteries where it might be awkward or impractical to insert a balloon-tipped catheter, or where the catheter atherotome is introduced into an artery percutaneously.

In yet another embodiment of the invention a stocking-like filter is attached to the catheter atherotome supported by the blades, to open and close as the blades are expanded or retracted. A guide wire extends through the lumen of the inner member of the catheter. The filter is attached to the distal end of the inner member of the atherotome to aid in closing the filter for retrieval.

In percutaneous use of the catheter atherotome of the invention a hollow needle is inserted through the skin into an artery. A guide wire is passed through the needle bore and directed to the point of stenosis in the arterial system. The needle is removed and a sheath/dilator is passed over the guide wire and the dilator is then removed from the sheath/dilator, leaving the sheath in place. Thereafter, the catheter atherotome is inserted into the artery over the guide wire and through the sheath into the artery and on to the point of stenosis. After appropriate excision of atherosclerotic plaque, the catheter atherotome and guide wire are removed and pressure is held at the point of skin puncture to permit closure of the artery to occur.

It is, therefore an important object of the present invention to provide an improved catheter atherotome for use in relief of stenoses in arteries.

It is another important object of the present invention to provide such a device which is manually adjustable between a configuration in which cutting blades are retracted and a configuration in which cutting blades are operatively positioned and exposed to a degree controllable by the user of the device.

It is an important feature of the apparatus of one embodiment of the present invention that its blades are slightly toed in but somewhat flexible, so that they may glide along the interior of elastic, healthy portions of an artery, without cutting. The blades evert upon encountering rigid plaque, so as to cut it.

The foregoing and other objectives, features, and advantages of the invention will be more readily understood upon consideration of the following detailed description of the invention, taken in conjunction with the accompanying drawings.

### Brief Description of the Drawings

FIG. 1 is a pictorial view of a catheter atherotome which embodies the present invention, with the catheter portion shown foreshortened, and with the basket knife in a retracted or closed position.

FIG. 2 is a sectional view of the catheter atherotome shown in FIG. 1, taken along the line 2-2 of FIG. 1, and simplified by omission of some of the blades of the basket knife.

FIG. 3 is a view similar to FIG. 2, with the basket knife in an expanded, or cutting configuration.

FIG. 4 is an edge-on view of one of the blades of the basket knife of the atherotome shown in FIGS. 1-3.

FIG. 5 is a side view of the blade shown in FIG. 4.

FIG. 6 is a sectional view, taken along line 6-6 of the blade shown in FIG. 5.

FIG. 7a is a perspective view showing a fitting for attaching the distal end of each blade to the end of the inner member of the catheter atherotome shown in FIGS. 1-3.

FIG. 7b is a perspective view of the fitting attaching the proximal ends of the blades to the distal end of the outer sheath portion of the catheter atherotome shown in FIGS. 1-3.

FIG. 8 is a schematic view showing the arrangement of the several blades of one embodiment of the catheter atherotome of the present invention.

FIG. 9 is a view similar to that of FIG. 8, of a set of somewhat different blades for the basket knife of the catheter atherotome of FIGS. 1-3.

FIG. 10 is a view similar to that of FIG. 8, schematically showing a third set of blades which embody the invention.

FIG. 11a is a pictorial view of the basket knife portion of a catheter atherotome of an alternate embodiment of the invention, including a skirt portion associated with the blades of the basket knife.

FIG. 11b is a detail view, at an enlarged scale, of the distal end of the catheter atherotome shown in FIG. 11a.

FIGS. 12a, 12b and 12c are sectional views of a portion of an artery including an atheroma, showing the action of the catheter atherotome of the present invention as it is drawn past the atheroma to remove a portion of the plaque material forming the atheroma.

FIG. 13 is a pattern view of a blade for a catheter atherotome according to the present invention which may be of sheet material, showing the blade in a flat condition.

FIG. 14 is a view similar to that of FIG. 13 showing a somewhat different blade.

FIG. 15 is a view similar to that of FIG. 13 showing yet another blade.

FIG. 16 is a view similar to that of FIGS. 13-15, showing a blade including a flexible body portion and a sharp edged insert.

FIG. 17 is a sectional view, taken along line 17-17 of FIG. 16.

FIG. 18 is a view similar to that of FIG. 16, showing a somewhat different blade including an insert.

FIG. 19 is a sectional view, taken along line 19-19 of FIG. 18.

FIG. 20 is a view similar to that of FIG. 16, showing a somewhat different blade member including an insert.

FIG. 21 is a sectional view, taken along line 21-21 of FIG. 20.

FIG. 22 is a perspective view of a fitting for attaching a terminal portion of each of a plurality of blades to the distal and proximal ends of a respective portion of the catheter atherotome according to the present invention.

FIG. 23 is a sectional view of a portion of the fitting shown in FIG. 22.

FIG. 24 is a side view of another type of blade useful in the catheter atherotome according to the present invention.

FIG. 25 is an axial view, taken in the direction indicated by the line 25-25, of the blade shown in FIG. 24.

FIG. 26 is a view of the blade shown in FIG. 24, taken along the line 26-26.

FIG. 27 is a pictorial view of a portion of a catheter atherotome according to the present invention including a filter attached to the blades of the atherotome.

FIG. 28 is a partially cut-away view, at an enlarged scale, of the portion of a catheter atherotome shown in FIG. 27.

FIG. 29 is a detail view, at a further enlarged scale, of a portion of one of the blades of the catheter atherotome shown in FIGS. 27 and 28.

FIG. 30 is a perspective view of a portion of a catheter atherotome in which blades are provided with tension bars.

FIGS. 31a, 31b and 31c are sectional views of a portion of an artery including an atheroma, schematically showing the action of the catheter atherotome shown in FIG. 30 as it is drawn past the atheroma to remove a portion of the plaque material of the atheroma.

### Best Modes for Carrying out the Invention

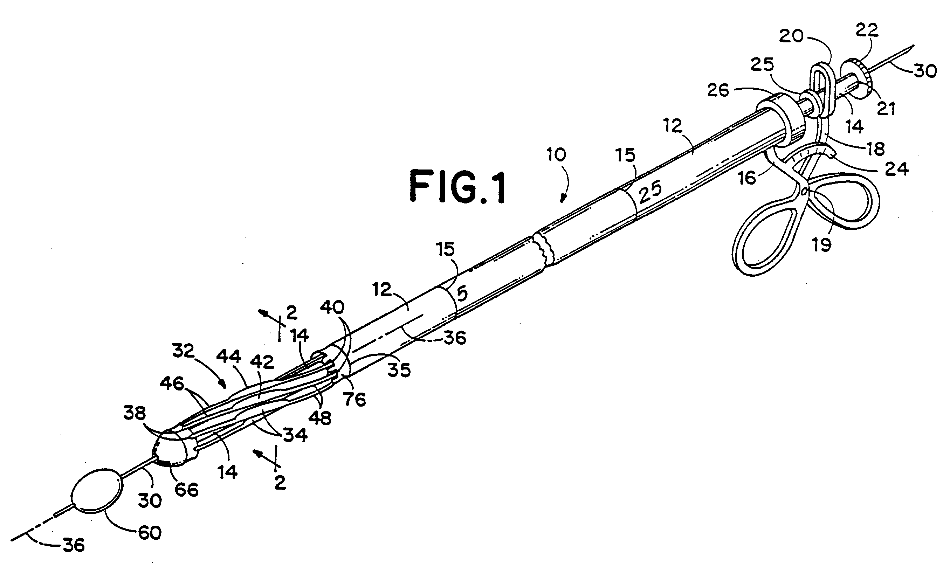

Referring now to the drawings which form a part of the disclosure herein, in FIGS. 1-3 a catheter atherotome 10 includes an elongate flexible tubular outer sheath 12. A similar inner member 14 of somewhat greater length is disposed within the outer sheath 12. The outer sheath 12 and inner member 14 are flexible enough to negotiate curves in arteries atraumatically, but are rigid enough to maintain relative position between the two, both longitudinally and rotationally. They might be made, for example, of a suitable polyvinyl chloride plastic material. Markings 15 may be provided along the outer sheath 12 to indicate the length of the catheter atherotome distal of each marking as an aid to placement in an artery.

A first lever 16 is connected with the outer sheath 12 near its proximal end. A second lever 18 is pivotally connected with the first lever 16 at a pivot joint 19. An elongate loop 20 is attached to the second lever 18 and surrounds the rear or proximal end portion 21 of the inner member 14. A stop 22 is fixedly attached to the proximal end of the inner member 14. The stop 22 is preferably larger than the loop 20 and is provided with appropriate surface configuration to permit rotation of the inner member 14 manually with respect to the outer sheath 12 by use of the stop 22. Finger loops are provided on the levers 16 and 18 for use in manipulation of the first and second levers 16 and 18 to withdraw the proximal end 21 of the inner member 14 a distance from the proximal end 23 of the outer sheath 12, as desired. A second stop 25 is preferably also located on the inner member 14, on the distal side of the loop 20, to be used, if necessary, to push the inner member 14 distally into the proximal end 23 of the outer sheath 12. Preferably, a scale 24 is provided on the first lever 16 as an indicator of the position to which the inner member 14 has been withdrawn relative to the outer sheath 12. A cap 26 is mounted on the proximal, or rear end of the outer sheath 12 by means of mating threads, and an O-ring 28, held in place by the cap 26, grips the exterior surface of the inner member 14 with an appropriately adjustable amount of force to maintain the position of the inner member 14 relative to the outer sheath 12.

The inner member 14 is tubular, with a lumen which is large enough to admit passage of a guide wire (not shown) or a balloon-tipped catheter 30 which may, for example, be a Fogarty® arterial embolectomy catheter.

A controllably expansible basket knife 32 includes a plurality of blades 34 extending between the distal end 35 of the outer sheath 12 and the distal end 37 of the inner member 14, which extends, as previously mentioned, beyond the distal end of the outer sheath 12. While six blades, equally spaced, are shown, fewer or more might also be utilized. The catheter atherotome 10 can be of an appropriate size, depending on the size of the artery, and larger blades 34 are required for use in larger arteries.

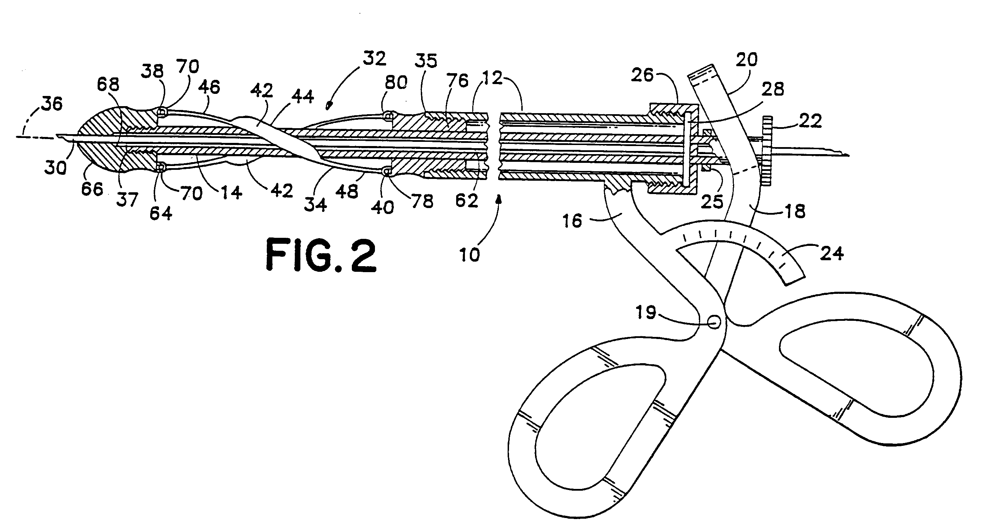

As shown in FIGS. 1 and 2, when the inner member 14 is located with its distal end 37 in a position of maximum extension beyond the distal end 35 of the outer sheath 12, the blades 34 extend closely alongside the protruding portion of the inner member 14, normally in a helical configuration centered about a central longitudinal axis 36. The distal ends 38 of the several blades 34 are separated from the positions of the proximal ends 40 of the blades 34, by an angle determined by the rotational position of the inner member 14 with respect to the outer sheath 12. Thus, rotation of the inner member 14 within the outer sheath 12 causes a steeper or shallower slope or pitch of each of the blades 34. Thus, as shown in FIG. 1, the distal ends 38 of the blades are located approximately 60° offset from the respective proximal ends 40, with the blades 34 extending generally longitudinally and closely alongside the inner member 14, in a steep helix.

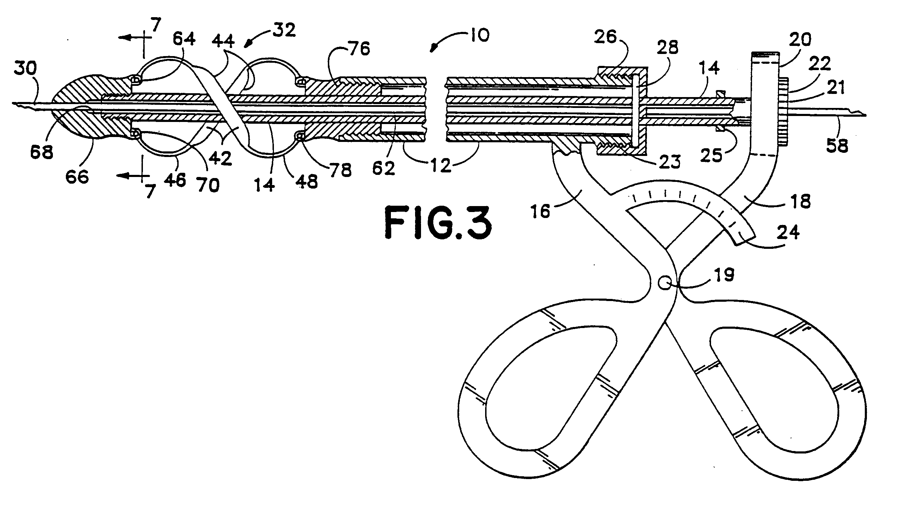

It should be recognized that FIGS. 2 and 3, for the sake of clarity, show but two of the blades 34, of which there would normally be a greater number, for example, six blades 34. It should further be recognized that the inner member 14 is shown in a position of rotation relative to the position of the outer sheath 12 in which the distal ends 38 are separated from the respective proximal ends 40 by about 180°. The degree of rotation shown is an exaggeration which probably would not be used in practice, but is used here in order to provide greater clarity in the figures for depiction of the effects of a combination of rotation and retraction of the inner member 14 with respect to the position of the outer sheath 12.

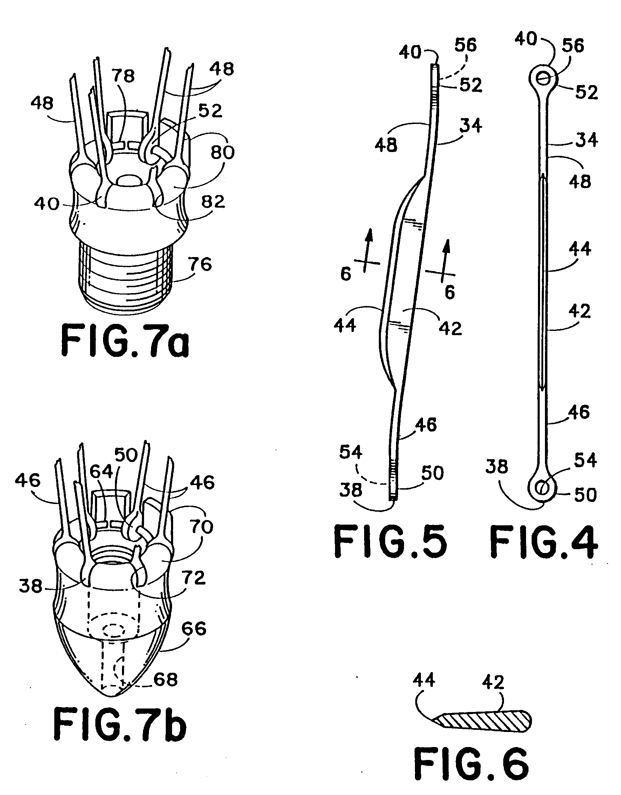

Referring now also to FIGS. 4, 5, and 6, it will be seen that each blade 34 includes a middle portion 42 including a sharpened edge 44, an intermediate, or strut, section 46, between the distal end 38 and the middle portion 42, and an intermediate, or strut, portion 48, located between the middle portion 42 and the proximal end 40. A pair of fins, a fin 50 proximate the distal end 38 and a parallel fin 52 proximate the proximal end 40 of the blade, respectively define bores 54 and 56. The fins 50 and 52 are oriented generally perpendicular to the orientation of a plane defined by the middle portion 42 of the blade, and the bores 54 and 56 extend generally normal to the fins 50 and 52, while the blades 34 are gently curved in an "S" shape when relaxed.

The catheter 30 extending through the catheter atherotome 10 has a balloon tip 60 which extends beyond the distal end of the catheter atherotome 10, as may be seen in FIG. 1. The catheter 30 is longer than the entire catheter atherotome 10, so that it can be manipulated at the proximal end of the catheter atherotome 10 while extending through a lumen 62 defined within the inner member 14, as shown in FIGS. 2 and 3. Such length is also desirable to provide room for cutting strokes of the atherotome with the balloon tipped catheter stationary in an artery.

The intermediate, or strut, portions 46 and 48 of each blade 34 are flexible in response to movement of the inner member 14 relative to the outer sheath 12, so that rotation of the inner member and retraction of the distal end 37 of the inner member with respect to the distal end 35 of the outer sheath result in flexure of the blades 34. Flexure takes place particularly in the strut portions 48 and 46 of the blades 34, to expand the plurality of blades 34 into a basket-like configuration in which each edge 44 is inclined more steeply with respect to the central longitudinal axis 36. While the strut portions 48 and 46 are free to bend relatively uniformly and to twist, the wider and more flattened shape of the sharpened middle portion 42 constrains its flexure to a degree which permits the edge 44 to assume an arcuate curvature centered about the inner member 14 but at a radius greater than that of the outer sheath 12, with the sharpened edge 44 directed toward the proximal end of the catheter atherotome 10. The inclination of the individual blades 34, and particularly the edge 44 thereof, is determined by both the degree to which the inner member 14 is withdrawn proximally within the distal end 35 of the outer sheath 12, and the degree of rotation of the inner member 14 with respect to the outer sheath 12. Preferably, the edge 44 can extend up to an angle of 40° to 50° relative to an imaginary equator of the basket knife 32 to achieve maximum cutting, with the equator considered to be normal to the longitudinal axis 36.

As may be seen with reference more particularly to FIGS. 7a and 7b, as well as FIGS. 2 and 3, the blades 34 are prevented from rotating with respect to the inner member 14 and outer sheath 12 by the manner in which they are attached. An articulating mounting ring 64 extends through the bores 54 of all of the several blades 34. The articulating mounting ring 64 is securely attached to a distal fitting 66, which has an ogival shape. The fitting 66 is attached to the distal end 37 of the inner member 14 by exterior threads defined on the inner member 14 and interior threads in the distal fitting 66. A bore 68 in the distal fitting 66 is an extension of the lumen 62 of the inner member 14.

The articulating mounting ring 64 is attached to the proximal end of the distal fitting 66 by a plurality of tethering hasps 70 disposed about the proximal end of the distal fitting 66 and equal in number to the number of blades 34 to be attached to the distal fitting 66. Each of the tethering hasps 70 is bent inwardly to form an arch over the articulating mounting ring. Adjacent ones of the several tethering hasps 70 cooperatively define radially extending slots 72 between one another, with the fin 50 of a respective one of the blades 34 being disposed within each of the slots 72. The slots 72 and fins 50 restrain each of the blades 34 from rotation about an axis parallel with the central longitudinal axis 36, but permit a terminal portion of each blade, including the fin 50 and the strut or intermediate portion 46, to pivot, about the articulating mounting ring 64 as the blade 34 is bowed.

A proximal blade fitting 76 is attached to the distal end 35 of the outer sheath 12 by exterior threads mated with interior threads defined in the outer sheath 12. An articulating mounting ring 78, similar to the articulating mounting ring 64, extends through the several bores 56 defined by the fins 52 of the blades 34, interconnecting all of the proximal ends 40 of the blades. The articulating mounting ring 78 is attached to the proximal fitting 76 by a plurality of tethering hasps 80 equal in number to the number of blades 34.

Similar to the tethering hasps 70, the tethering hasps 80 extend from the distal face of the proximal fitting 76 and are bent arcuately inward toward the central longitudinal axis 36 of the catheter atherotome 10, arching over and retaining the articulating mounting ring 78. The tethering hasps 80 define radially extending slots 82 between adjacent ones of the hasps 80 and restrict the proximal ends 40 of the several blades 34 from rotating about an axis parallel with the central longitudinal axis 36 of the catheter atherotome 10. Terminal portions of the blades 34 located adjacent the proximal ends 40 are free to pivot with the fins 52 rotating about the articulating mounting ring 78 in response to retraction of the distal end of the inner member 14 into the distal end of the outer sheath 12 to the position shown, for example, in FIG. 3.

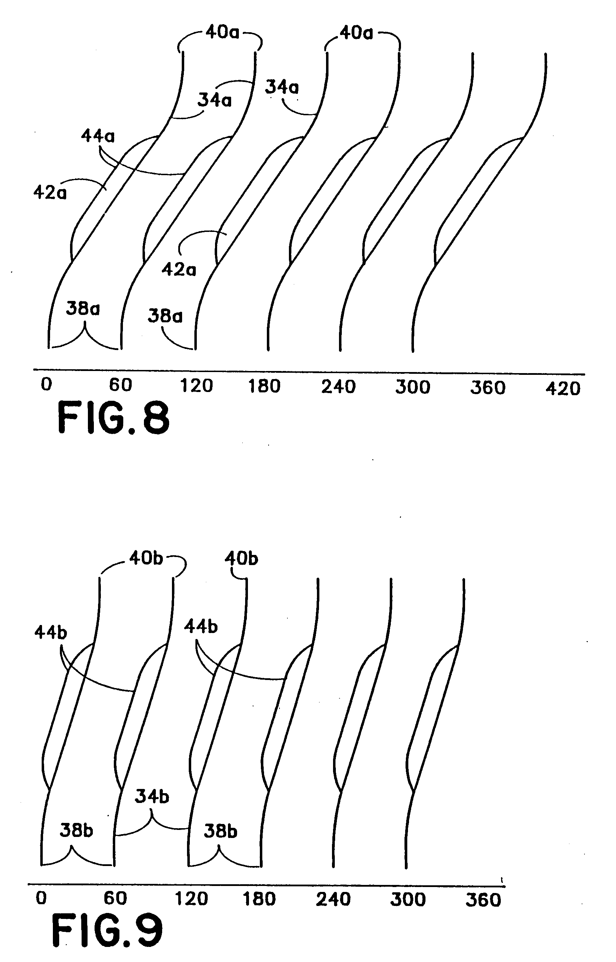

Referring to FIGS. 8, 9, and 10, the configuration and degree of flexibility of a set of blades 34 may be made to provide different amounts of coverage of the circumference of the interior or lumen of an artery within which partial atherectomy is to be performed.

In FIG. 8, six blades 34a for use as part of the catheter atherotome 10 according to the present invention are shown in a simplified schematic form as if the articulating mounting rings 64 and 78 had been straightened and unwrapped from around the circumference of the inner member 14, while maintaining the circumferential spacing of the blades 34a so as to show the angular relationships between their respective opposite ends. It will be seen, then, that the angular distance of the distal ends 38a from the proximal ends 40a is greater than 60°. Thus, the distal end of one of the blades 34a is offset angularly beyond the proximal end 40 of the adjacent blade 34a, and the middle portions 42a of adjacent blades 34a also overlap one another so that the edges 44a cooperatively present a substantially continuous circular combination of cutting edges 44a to remove a layer of plaque from the entire interior surface of the atheroma being reduced on each cutting stroke of the atherotome. The blades 34a preferably are stiff enough so that the angle of incidence of the cutting edge is fairly stable, so that the cutting edge can pare off a thin slice of atherosclerotic plaque or similar material from the interior of an artery during use of the catheter atherotome 10 equipped with the blades 34a.

Referring now more particularly to FIG. 9, a set of blades 34b are shown which have less offset between the locations of their distal ends 38b and their proximal ends 40b, so that the cutting edges 44b make separate cuts which do not overlap one another ordinarily. However, the blades 34b are made with greater flexibility and less torsional rigidity than the blades 34a. This is so that, although the angle of incidence of the edges 44 ordinarily is too little to result in cutting the interior of the arterial wall, when the sharp edge 44b of an individual blade 34b "snags" on a hard irregular body of plaque the blade is everted into a cutting position with a somewhat greater angle of incidence in order to excise that piece of plaque, as will be explained more fully subsequently. Because of the lesser offset between the distal and proximal ends of the blades 34b there is an interrupted coverage of the circumference of an artery, providing space for irregular protrusions of plaque to project between the blades 34b to be engaged by the sharpened edges 44b.

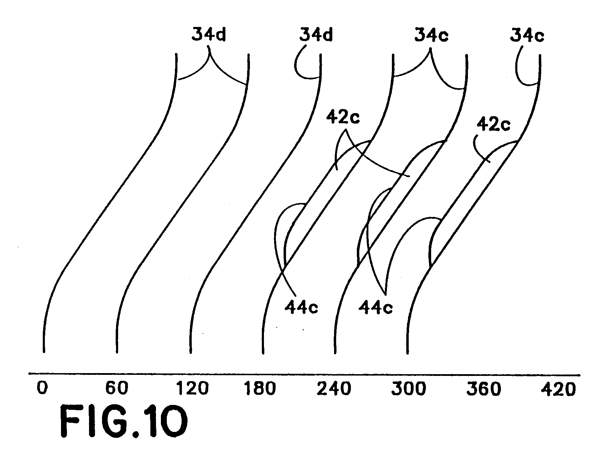

In FIG. 10 is shown a set of blades 34c and 34d, of which only the three blades 34c include middle portions 42c having edges 44c, while the blades 34d have no sharpened edge portions. Thus, the set of blades 34c and 34d provide cutting edge coverage over only about one-half the interior circumference of an artery and are most useful in removing plaque from the posterior side of an artery such as the femoral artery, where atheroma normally occurs only on the posterior side of the artery. In some situations it may be desirable to include a sharpened edge 44c on only a single one of a set of blades 34c and 34d. The blades 34d without sharpened edges then help to maintain the proper relationship between the blades 34c, keep the basket knife apparatus centered within the arterial lumen, and provide some tension in the arterial walls.

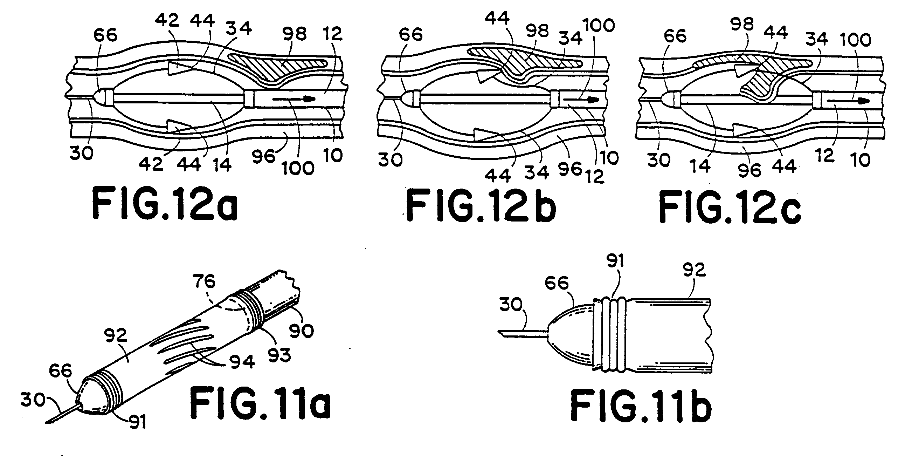

In some instances a catheter atherotome may be required to be of a size that is too small to admit passage of the balloon-tipped catheter 30 therethrough. It is still necessary to be able to retrieve pieces of plaque which have been cut free from the interior wall of an artery by use of the atherotome. In order to recover the matter excised from an arterial wall, a catheter atherotome 90, shown in FIGS. 11a and 11b, which is otherwise similar to the catheter atherotome 10, additionally includes a flexible tubular membrane in the form of a skirt 92 arranged about and attached to the blades 34. One end of the skirt is attached to the distal fitting 66 by means of ferrules 91 and the other end is attached to the proximal fitting 76 by means of ferrules 93. The skirt 92 is preferably adherently attached to the blades 34, at least along the middle portions 42, and slits 94 or equivalent openings are provided to expose the edges 44 and provide ingress for pieces of plaque to the interior of the skirt 92. Pieces of plaque or the like cut free from the interior wall of an artery are able to pass through the slits 94 to be collected upon retrieval of the catheter atherotome 90 from within an artery. The membrane used as the skirt 92 must be flexible and thin, yet strong and elastic enough to accommodate the adjustment of the basket knife 32. A suitable material is a thin sheet of latex. The skirt 92 may be attached to distal fitting 66 and the proximal fitting 76 after assembly of the catheter atherotome 10 including the blades 34.

Referring now to FIGS. 12a, 12b, and 12c, showing a portion of an artery 96 including an atheroma 98, the catheter atherotome 10 is illustrated schematically to show its use. As shown in FIG. 12a, the catheter atherotome 10 has been inserted into the artery from right to left. Thereafter, the inner member 14 has been withdrawn a distance into the distal end 35 of the outer sheath 12, so that the blades 34 are bowed, to place the middle portion 42 of each blade at an increased radial distance away from the inner member 14 and to cause the sharpened edges 44 to come into proper position.

To excise a portion of the atheroma 98, the catheter atherotome is manually pulled to the right as shown in FIG. 12b, and as indicated by the arrow 100. As the inwardly projecting atheroma 98 is encountered by the upper blade 34, and particularly by the edge 44, engagement of the edge 44 in the atheroma plaque material causes the edge 44 to be everted into a cutting position in which it digs into the plaque material and begins to cut a portion of the plaque material free, as may be seen in FIG. 12c. Torsional forces developed in the strut portions 46 and 48 control the angle of incidence of the edge 44, allowing the middle portion 42 to be moved to such a cutting position when plaque is encountered. However, where there is no plaque material present, as in the lower portions of the artery shown in FIGS. 12a, 12b, and 12c, the edge 44 of the respective blade 34 is oriented so that it does not catch the intima of the arterial wall and simply slides along the interior wall of the artery without doing any cutting.

The surgeon using the catheter atherotome 10 can repeatedly move the basket knife 32 back and forth in the area of an atheroma such as the atheroma 98, cutting away a thin layer of plaque with each pass in the direction indicated by the arrow 100 in FIGS. 12b and 12c, until the lumen of the artery 96 has been opened sufficiently. After each cutting stroke the entire catheter atherotome 10 will be rotated through an angle which can be determined by the position of the levers 16 and 18, so as to result in excision of plaque in an evenly distributed pattern about the interior of the artery. In some situations it will also be desirable to rotate the atherotome as it is being drawn proximally. Thereafter, the inner member 14 may be allowed to resume its extended position beyond the distal end of the outer sheath 12, using the loop 20 (see FIG. 1) against the second stop 25 if necessary, thus retracting the middle sections 42 of the blades 34 closer to the inner member 14. The catheter atherotome 10 can then be withdrawn from the artery, followed by withdrawal of the balloon-tipped catheter 30, with the balloon 60 (see FIG. 1) inflated to retrieve the material which has been cut free from the arterial wall during the process.

The procedure is similar when using the catheter atherotome 90 (FIG. 11a), except that the material cut free from the arterial wall would be retained within the membrane skirt 92 for retrieval along with the atherotome 90 when it is withdrawn from the artery.

The catheter atherotome 10 may be introduced into an artery including a stenosis by providing access to the artery and opening the arterial wall at a position more proximal to the heart than the location of the stenosis. Preferably, a guide wire is introduced into the artery and past the stenosis. Thereafter, if required, a dilator may be introduced into the artery, guided by the wire. The dilator may then be withdrawn and the catheter atherotome 10 according the present invention may be inserted into the artery along the guide wire, which may then be withdrawn and replaced by the balloon-tipped catheter 30. After inflation of the balloon 60 to prevent loss of pieces of material cut free from the arterial wall by the atherotome 10, the basket knife 32 may be expanded to the required size by withdrawal of the inner member 14 into the distal end 35 of the outer member 12 a required distance by squeezing together the finger loops of the levers 16 and 18, so that the elongate loop 20 acts upon the stop 22 to withdraw the proximal end 21 of the inner member 14 at the proximal end of the catheter atherotome 10. Preferably, the scale 24 provided on the lever arm 16 may be used to determine when the inner member 14 has been withdrawn sufficiently to provide the required expansion of the basket knife 32.

Thereupon, the catheter atherotome may be withdrawn past the location of the atheroma, with the sharpened edges 44 of the blades 34 of the basket knife 32 paring away a portion of the plaque from the interior of the artery. The atherotome is then pushed into the artery until the blades 34 are beyond the atheroma, and rotated to a desired position for a subsequent pull-back cutting stroke. After several strokes, for example, six to ten cutting strokes, sufficient enlargement of the lumen of the artery should have taken place, and the basket blade 32 can be relaxed, contracting the blades 34 into position alongside the inner member 14 as shown in FIG. 1, so that the atherotome can be withdrawn from the artery.

Objective confirmation of enlargement of the arterial lumen by the use of the catheter atherotome could be obtained either by on-the-table angiography, for comparison with the pre-operative arteriogram, or an on-the-table duplex-ultrasound measurement could be made of the arterial lumen immediately before and after use of the catheter atherotome of the present invention.

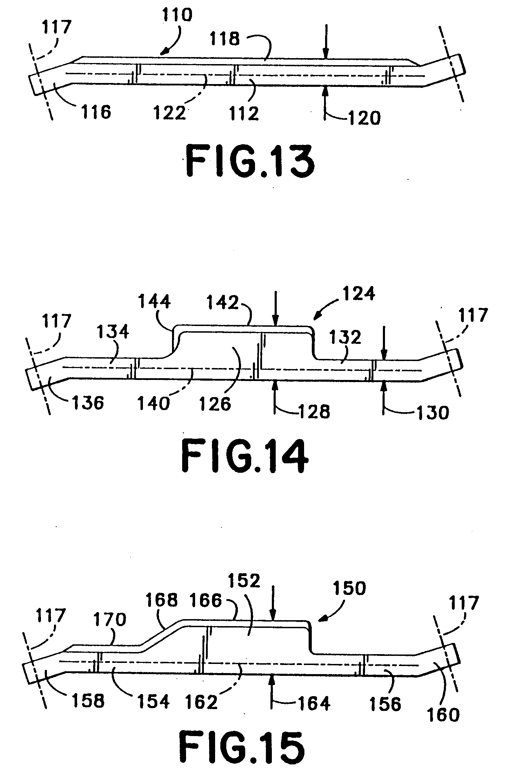

Referring now also to FIGS. 13-15, blades for the basket knife of a catheter atherotome 10 may be manufactured by cutting the appropriate shape from a thin sheet of appropriately resilient and strong metal. For example, a blade 110 has a middle portion 112 and a pair of opposite terminal portions 114 and 116. The middle portion 112 defines a sharp edge 118 which is essentially, straight and extends over substantially the entire middle portion 112, being beveled, as by grinding, on either one or both sides of the middle portion 112. The middle portion 112 has a width 120, while the terminal portions 114 and 116 are somewhat narrower, so that they can be attached as will be explained subsequently. The terminal portions 114, 116 of the blade 110 will be bent to form attachments and will be able to pivot about axes indicated at 117, as will be understood presently.

As shown in FIG. 13, with the blade 110 in a planar configuration, the terminal portions 114 and 116 extend at an acute angle to the central longitudinal axis 122 of the middle portion 112. Since the terminal portions 114, 116 are slightly offset, they can be attached to the respective fittings of the catheter atherotome to extend part of the way around the catheter atherotome in a generally helical configuration, with an angular offset, about the longitudinal central axis of the catheter atherotome, between the distal and proximal ends of the blade. Because the width 120 is uniform throughout the entire middle portion 112, the blade 110 tends to have a uniform curvature when in place on a catheter atherotome according to the present invention.

The blade 124 shown in FIG. 14 includes a middle portion 126 having a width 128 which is greater than the width 130 of the intermediate portions 132, 134 located adjacent the middle portion 126, on either end thereof. Terminal portions 136, 138 may have widths similar to the widths 130, but are preferably oriented angularly slightly offset from the central longitudinal axis 140 of the intermediate portions 132, 134, so that the blade 124 can be attached with the terminal portion 132, 134 angularly offset from each other about an inner member 14 of a catheter atherotome according to the invention. The middle portion 126 extends to one side of the blade 124 and includes a sharp edge 142. The sharp edge 142 extends parallel with the central axis 140 and also includes a proximal portion 144 which is to be located at the proximal end of the catheter atherotome's basket knife assembly, that is, so that the terminal portion 136 is attached to the fitting attached to an outer sheath member 12 of the catheter atherotome.

Referring to FIG. 15, a blade 150 includes a middle portion 152, a proximal intermediate portion 154, a distal intermediate portion 156, and respective terminal portions 158 and 160. As in the blade 124, shown in FIG. 14, the middle portion 152 extends on one side of the central longitudinal axis 162 of the intermediate portions 154 and 156, defining a width 164 of the middle portion 152. A sharp edge 166 extends along the middle portion 152, on the side thereof which protrudes laterally beyond the intermediate portions 154 and 156. The sharp edge 166 continues as a diagonal portion 168 and a portion 170 which extends along the intermediate portion 154, which is normally located close to the proximal end of the catheter atherotome, to be exposed to be useful in cutting away an atheroma as the catheter atherotome is drawn proximally by the user.

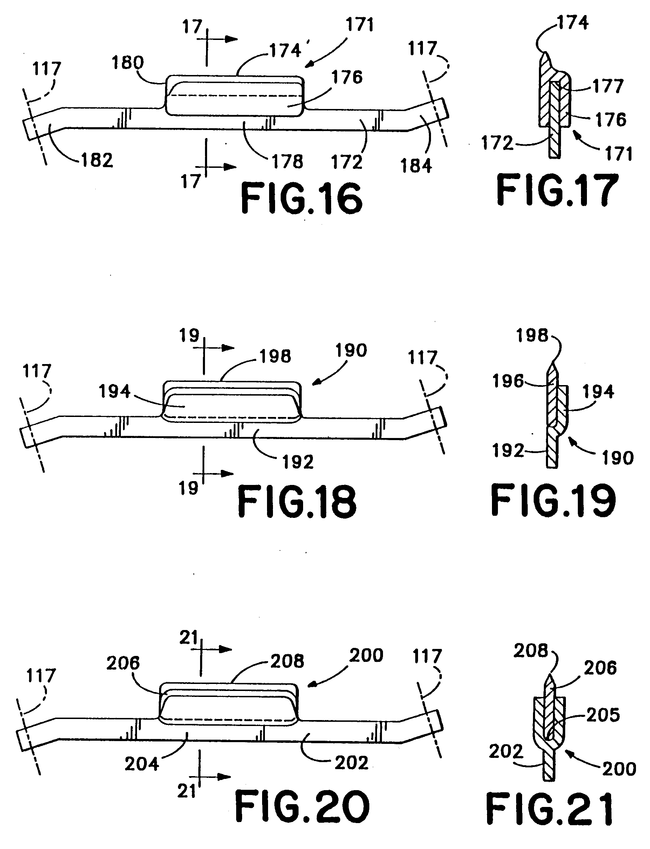

Referring now to FIGS. 16 and 17, a blade 171 is generally similar in shape to that of the blade 124 shown in FIG. 14, but rather than being made of a single piece of metal, the blade 171 may have a body 172 of either metal or another suitably strong and resiliently flexible material such as a suitable molded plastic, with a sharpened edge 174 being defined on a separate insert piece 176 attached to the main body 172 by suitable means such as an adhesive (not shown). As shown in FIG. 17, the sharpened piece 176 may define a groove 177 to receive a portion of the main body 172, and the sharpened edge 174 may continue onto the proximal end of the middle portion 178 of the blade 171, as at 180. The blade 171 includes respective proximal and distal terminal portions 182, 184, which are preferably oriented in an angularly offset direction as in the previously-described blades 110, 124, and 150, for the same reason.

Referring to FIGS. 18 and 19, a blade 190 is similar to the blade 171 except that the main body portion 192 includes an offset portion 194. A sharpened insert 196 with a sharpened edge 198 is attached on one side of the offset portion 194, as shown in FIG. 19, where it is retained by suitable means such as an adhesive material.

As shown in FIGS. 20 and 21, yet another blade 200 includes a main body portion 202 having a middle portion 204 having a pair of parallel members defining a channel 205 within which a sharpened edge insert 206 is held, as by an adhesive material. A sharpened edge 208 is located similarly to the location of the sharpened edge 198 and the sharpened edge 174 in the blades 190 and 170, respectively. In each of the blades 124, 150, 170, 190, and 200, the width of the middle portion of the blade, being greater than the widths of the intermediate portions of the blade, results in greater flexure of the intermediate portions of the blades in response to movement of the inner member relative to the outer sheath of the catheter atherotome, in either a longitudinal direction or in rotation, while the sharp edge portions 142, 144, 166, 168, 174, 198, and 208 retain a predetermined radius of curvature.

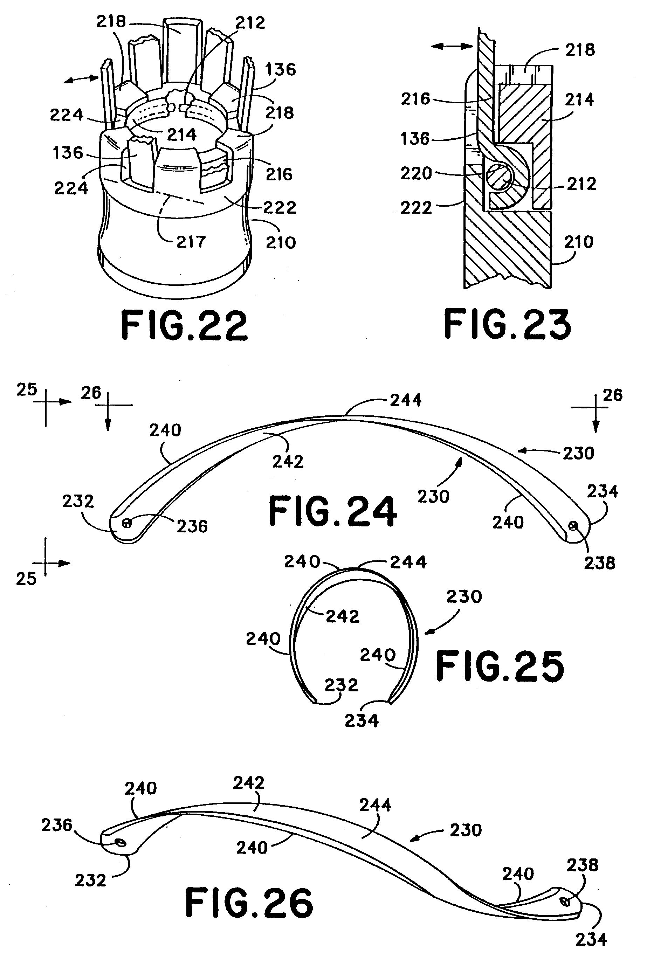

A fitting 210 shown in FIGS. 22 and 23 may be fashioned of metal in a form which can be attached respectively either to the distal end of the outer sheath 12 or the distal end of the inner member 14 of a catheter atherotome to support the respective ends of blades such as the blades 110, 124, etc. The fitting 210 receives the terminal portions of such blades and holds them in a manner permitting the blades to flex and become outwardly bowed and permits rotation of the inner member relative to the outer sheath of the catheter atherotome so as to adjust the location and pitch of the sharp edge portions of the blades. An articulating mounting ring 212 may be formed of suitable material such as an appropriately strong wire or an appropriately strong plastic material, for example. A retainer ring 214 defines a number of notches 216 preferably equal to the number of blades to be retained and rests atop the articulating mounting ring 212. A plurality of tethering hasps 218 which are integral with the main body of the fitting 210 are bent over to extend radially inward atop the retainer 214 to hold it in place, retaining each of the blades. The terminal portions, such as the terminal portions 136, 138, etc. of the respective blades, are formed to define a generally U-shaped channel 220, within which the articulating ring 212 rests in a location radially outward of the surface of the respective terminal portion. The articulating retaining ring 212, in turn, sits radially inward within an upstanding annular wall portion 222 of the body of the fitting 210, as shown in section view in FIG. 23. Thus the space between the tethering hasps 218 is available as a respective notch 224 within which the blades are free to pivot about the axis 117 defined by the respective portion of the wire articulating retaining ring 212 and the notches 216 defined in the retainer 214.

Another blade, similar in some respects to the blade shown in FIG. 13, is shown in FIGS. 24, 25, and 26. The blade 230 is preferably attached with respective proximal and distal terminal portions 232, 234 extending radially and attached to a retaining articulating ring (not shown) which may extend through respective bores 236, 238 defined in the proximal and distal terminal portions, respectively. Thus, a proximal portion of the sharp edge 240 extends radially with respect to the catheter atherotome at a location proximate the terminal portion 234. The middle portion 242 of the blade extends substantially the entire distance between the terminal portions 232, 234, with the sharp edge 240 extending along the entire middle portion 242. The middle portion 242 is twisted, however, so that the sharp edge 240, at the distal terminal portion 232 is located so that it is directed radially inward toward the inner member of the catheter atherotome where the distal terminal portion 234 of the blade 230 is attached to a fitting connecting the blade 230 with the inner member of a catheter atherotome. Preferably, the location of attachment of the terminal portion 232 is offset by an angle 241 about the central axis of the catheter atherotome, with respect to the location of the attachment of the terminal portion 234, so that the sharpened edge 240 curves, as shown in FIG. 25, in axial view along the catheter atherotome. Thus, the proximal leading portion of the sharpened edge 240 (closest to the proximal end of the catheter atherotome cutting assembly) is available to cut radially outward through a portion of a deposit of plaque while the portion of the sharpened edge 240 located more centrally of the length of the blade 230, as at 244, extends generally tangentially along the outermost limits of the shape of the basket knife of the atherotome, and the more distal portion of the sharpened edge 240 is inverted so that the blade 230 will cut its own way free from the wall of an artery as the catheter atherotome is drawn clear of a stenosis, in a proximal direction.

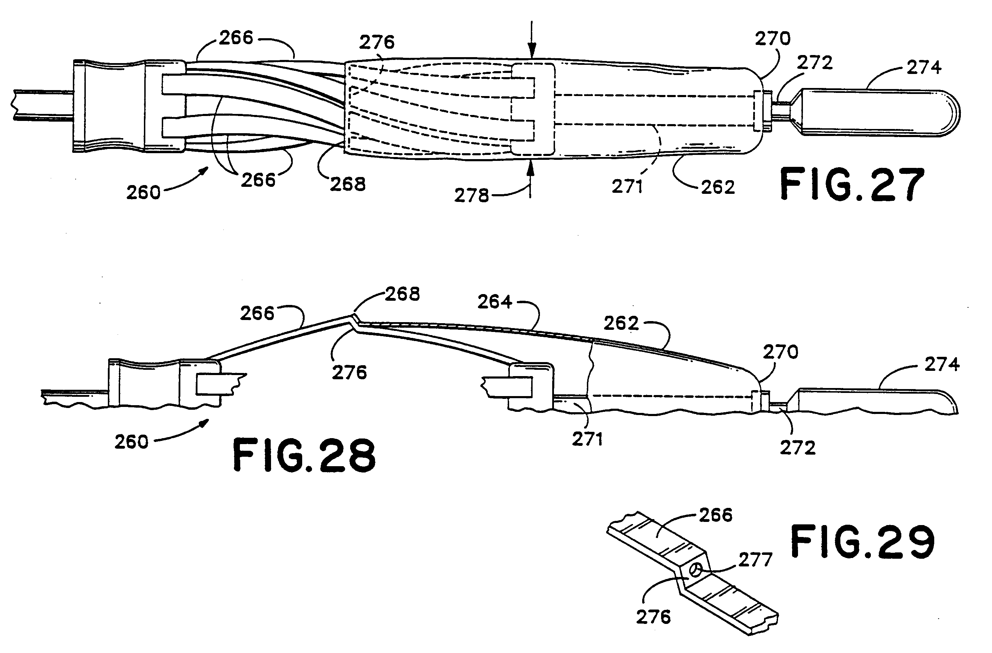

In FIGS. 27 and 28, a catheter atherotome 260 generally resembling the catheter atherotome 10 is equipped with a filter 262 in the form of a stocking-like generally tubular filter membrane 264 of an appropriate material such as a woven polyester fabric defining suitably small openings, i.e., openings which are small enough not to permit passage of particles large enough to obstruct smaller arteries or capillaries, while still permitting normal blood cells to proceed through the filter membrane 264. A proximal end, or mouth, 268 of the filter 262 is attached to the blades 266 of the catheter atherotome 260. A distal end 270 of the filter membrane 264 is securely and sealingly attached to the distal end 271 of the inner member of the catheter atherotome 260. A distal end of a guide wire 272, preferably equipped with a flexible tip 274, extends through a lumen defined in the inner member of the catheter atherotome 260 and beyond the distal end 270 of the filter membrane so that the guide wire 272 can be used to guide the catheter atherotome 260 to an atheroma, in a fashion similar to the use of a guide wire or the balloon catheter 30, as described with respect to the catheter atherotome 10.

As shown in FIG. 29, the mouth 268 of the filter 262 is attached at respective locations to each of the blades 266, where each blade includes an offset 276 such as an inwardly directed step formed in the blade 266. A bore 277 is provided, adjacent to the offset 276 to permit the proximal end, or mouth, of the filter 262 to be attached, as by being sewn or similarly attached to each blade at the corresponding point along the mouth 268 of the filter.

With the atherotome expanded as shown in FIG. 28 (where, for the sake of greater clarity, the blade 266 is shown without any offset angle about the central axis of the catheter atherotome, between the distal and proximal ends of the blade 266), the filter 262 is expanded diametrically to fill the lumen of an artery in which atherectomy is being performed, and particles of plaque cut free from the arterial wall are trapped within the filter 262 as the catheter atherotome is drawn proximally in the process of performing atherectomy.

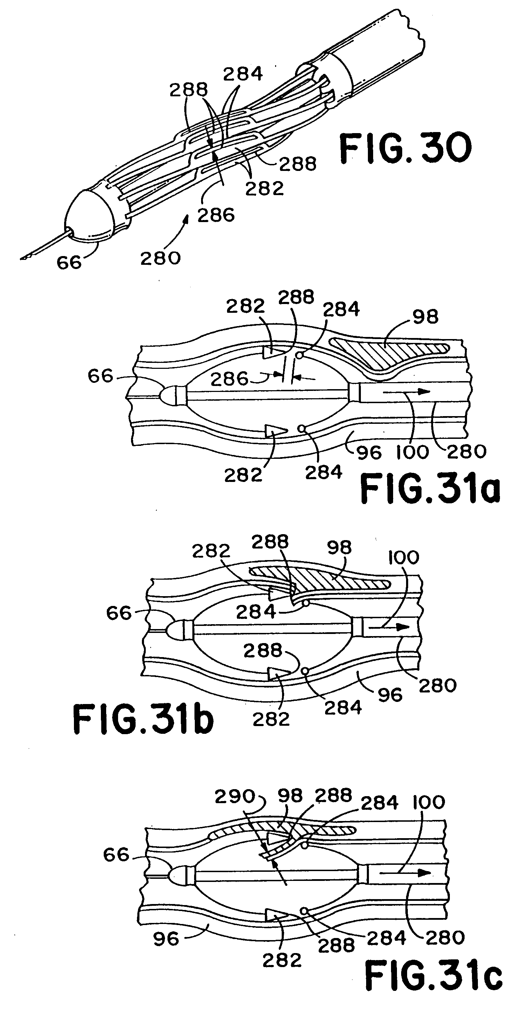

In a further embodiment of the invention a catheter atherotome 280, shown in FIGS. 30 and 31a, 31b and 31c, includes a plurality of blades 282 which include a tensioning bar 284 located alongside, generally parallel with, but spaced a small distance 286 apart from each sharp edge 288, in order to stabilize the surface of the atherosclerotic tissue as it is pared away by cutting strokes of the catheter atherotome. As shown in FIGS. 31a, 31b and 31c, where the same reference numerals are used as were used in FIGS. 12a, 12b and 12c to refer to the same items, the spacing distance 286 between the tensioning bar and the sharp edge will limit the thickness 290 of a piece of tissue cut free from an atheroma and should produce less trauma at the freshly cut atherectomy site, resulting in better healing and a lesser likelihood of recurrent occlusion. The tensioning bar 284 can be made, for example, as an integral part of the blade 282 as it is cut from a piece of sheet metal, by forming a fenestration and sharpening the edge 288 thereafter.

## Claims

1. A catheter atherotome (10) for use in surgical removal of plaque from the interior wall of an artery, comprising:
(a) an elongate tubular outer sheath (12) having respective proximal and distal ends (23 and 35);
(b) an elongate tubular inner member (14) disposed within said outer sheath and having respective proximal and distal ends (21, 37), said distal end of said inner member extending beyond said distal end of said outer sheath;
(c) a plurality of elongate blades (34) each having a proximal end (40) interconnected with said outer sheath (12) and a distal end (38) interconnected with said inner member;
(d) means (16, 18) for moving said inner member (14) with respect to said outer sheath (12) so as to retract said distal end of said inner member toward said distal end of said outer sheath and flex said blades into a radially outwardly bowed arcuate configuration; and
(e) at least some of said blades defining respective curved sharpened edges (44), at least a portion of each said curved sharpened edge extending along said inner member and being oriented more toward said proximal end than toward said distal end of said inner member when said blades are in said outwardly bowed arcuate configuration, so as to engage plaque (98) associated with an inner wall of an artery cuttingly as said catheter atherotome (10) is moved within said artery in a proximal direction, without engaging said interior wall of said artery cuttingly as said catheter atherotome (10) is moved within said artery in a distal direction, at least some of said curved sharpened edges (44) having respective opposite first and second ends (38, 40), said first and second ends (38, 40) thereof being angularly separated from each other by a controllably variable angle about said inner member (14) when said blades (34) are in said outwardly bowed arcuate configuration.

2. The catheter atherotome (10) of claim 1 wherein said inner member (14) defines lumen means (62) for receiving a guide wire extending through said inner member (14) for guiding said catheter atherotome (10) within said artery.

3. The catheter atherotome (10) of claim 1 wherein said means (16, 18) for moving said inner member (14) includes lever-operated means (16, 18) for moving said inner member (14) longitudinally with respect to said outer sheath (12).

4. The catheter atherotome (10) of claim 1 wherein said means (16, 18) for moving said inner member (14) includes means (20) associated with said inner member (14) for rotating said inner member (14) with respect to said outer sheath (12) through a limited angular distance, so as to vary said controllably variable angle about said inner member (14) and alter the inclination of said curved sharpened edges (44) with respect to said inner member (14) and thus with respect to a stenotic lesion (98) to be cut by said catheter atherotome (10).

5. The catheter atherotome (10) of claim 1 wherein each of said elongate blades (34) includes flexible portions (46, 48) thereof which do not define an edge thereof.

6. The catheter atherotome (10) of claim 1 wherein the ones of said blades (34) arranged over approximately one-half the circumference of said outer sheath (12) and inner member (14) include said sharp edges (44) while the remainder (46, 48) of said blades are free from said sharp edges.

7. The catheter atherotome (10) of claim 1 wherein each of said blades (34) defining a curved sharpened edge (44) includes a middle portion (42) defining said curved sharpened edge (44) and a pair of intermediate portions (46, 48) adjacent said middle portion, said intermediate portions (46, 48) being sufficiently more flexible than said middle portion (42) that said middle portion (42) substantially retains its original shape during flexing of said blades (34) into said radially outwardly bowed arcuate configuration.

8. The catheter atherotome (10) of claim 1 wherein each of said blades (34) has a terminal portion (38, 40) proximate to each end thereof and said inner member (14) defines a central longitudinal axis, the catheter atherotome (10) including means (70, 72) for preventing at least one of said ends of each of said blades from rotating with respect to said outer sheath (12) and said inner member (14) about an axis extending parallel with said central longitudinal axis, while permitting a respective one of said terminal portions (38, 40) of each of said blades (34) to rotate in a radial plane including said central longitudinal axis, between a position in which said terminal portion extends parallel with said longitudinal axis and a position in which said terminal portion extends away from said longitudinal axis at a larger acute angle and said middle portion of said blade is displaced radially further away from said longitudinal axis.

9. The catheter atherotome (10) of claim 8 including respective mounting means (Figures 22, 23) attached to at least one of said outer sheath (12) and said inner member (14) for attachment of said blades (34) to said inner member (14) and said outer sheath (12), each said mounting means (Figures 22, 23) including a pivot ring (212), and a respective terminal portion (136) of each of said blades (34) being bent to define channel means (220) for receiving a respective portion of said pivot ring (212).

10. The catheter atherotome (10) of claim 1 wherein each of said curved sharpened edges (44) of said blades (34) is located in a cutting position exposed toward said proximal end (23) of said outer sheath (12) and extending generally circumferentially about said inner member (14) when said distal end (37) of said inner member (14) is retracted toward said distal end (35) of said outer sheath (12) and said blades (34) are in said outwardly bowed arcuate configuration.

11. The catheter atherotome (10) of claim 1 wherein said blades (34) extend generally helically and said sharpened edges (44) extend generally helically and are exposed toward said proximal end (23) of said outer sheath member (12) when said blades (34) are in said outwardly bowed configuration.

12. The catheter atherotome (10) of claim 1 wherein said inner member (14) is tubular, further including balloon-tipped catheter means (30), disposed within the extending through said inner member (14) and having an inflatable balloon tip (60) located distally beyond said distal end of said inner member (14) and blades (34), for retaining and retrieving pieces of material cut free from an athermome (98) during use of said catheter atherotome (10).

13. The catheter atherotome (10) of claim 1, said plurality of blades (34) defining a basket (32) when said distal end (38) of said inner member (14) is retracted toward said distal end (35) of said outer sheath (12), said basket (32) defining an equator portion, and said respective ones of said curved sharpened edges (44) of said blades (34) being located along said equator portion of said basket (32) and being oriented generally helically about said outer sheath (14) of said catheter atherotome.

14. The catheter atherotome (10) of claim 1 wherein each of said blades (34) includes a pair of opposite intermediate portions (46, 48) and a middle portion (42), said intermediate portions being resiliently flexible and twistable, and at least some of said middle portions including respective ones of said curved sharpened edges (44), each said curved sharpened edge (44) normally being oriented helically about a central longitudinal axis of said inner member (14), and defining an angle of incidence, said angle of incidence being shallow enough so that said curved sharpened edge (44) ordinarily will not cuttingly engage the surface of the interior tissue of a healthy artery but being great enough so that said curved sharpened edge (44) will cuttingly engage atherosclerotic plaque (98) projecting from the interior surfaces of an artery, said intermediate portions (46, 48) being torsionally elastic enough to permit said middle portions (42) of said blades (34) to rotate resiliently with respect to said intermediate portions (46, 48) to a position in which said sharp edges (44) can engage and pare away a portion of said plaque material (98) from said interior surfaces of said artery.

15. The catheter atherotome of claim 1, further including means (262) for collecting shavings of material cut free from atheroma (98) by said blades (34).

16. The catheter atherotome (10) of claim 1 wherein some of said blades (34) are free from sharp edges and are located opposite said blades (34) defining curved sharpened edges (44), with respect to said inner member (14), so as to contact an interior surface of an artery and distend said artery when said blades (34) are in said radially outwardly bowed arcuate configuration.

17. The catheter atherotome (10) of claim 1 wherein at least one of said blades (34) is of a ribbon-like shape (42), having a leading margin (forward part of 44) and a trailing margin (trailing part of 44), said leading margin including a sharp edge, said proximal and distal ends of said blade further including respective terminal portions (40,50), and each said terminal portion defining respective fin means (40,50) for attaching said terminal portion to the respective one of said outer sheath (12) and said inner member (14).

18. The catheter atherotome (Figure 16) of claim 1 wherein at least one of said blades (171) has a body (172) of molded material and said sharp edge (174') is located on an insert (176) attached thereto.

19. The catheter atherotome (Figures 30, 31a, 31b, and 31c) of claim 1 wherein at least one of said blades (282) is of sheet metal and has a middle portion (282) and a pair of opposite terminal portions, said terminal portions being angularly offset with respect to said middle portion (282).

20. The catheter atherotome (Figure 20) of claim 1 wherein at least one of said blades (200) includes a body portion (202) of relatively flexible structure and a less-flexible, sharp-edge insert portion (206) fixedly attached to said body portion (202) and defining a respective one of said sharp edges (208).

21. The catheter atherotome (Figure 14) of claim 1 wherein at least one of said blades (124) includes a pair of opposite terminal portions (136) and a middle portion (126) extending therebetween, said middle portion (126) including a sharpened edge (142) extending between said terminal portions (136), said sharpened edge (142) including a leading portion (144) directed substantially radially with respect to said outer sheath (12).

22. The catheter atherotome (260) of claim 1 wherein said inner member (271) defines a lumen (62) and said second end (270) of said filter (262) is attached to a distal end of said inner member (14).

23. The catheter atherotome (Figures 20, 31a, 31b and 31c) of claim 1 including a tensioning member (284) extending parallel with the spaced apart from said sharp edge (288) of one of said bales (282) providing complete circumferential coverage of said artery so as to cut through a stenotic lesion (98) as said catheter atherotome (10) is moved in the proximal direction in said artery.

24. The catheter atherotome (10) of claim 1 wherein all of said blades (34) include respective curved sharpened edges (44) overlapping one another about said inner member (14) when said blades (34) are in said outwardly bowed arcuate configuration and said inner member (14) is in a predetermined angular position with respect to said outer sheath (12), providing complete circumferential coverage of said artery so as to cut through a stenotic lesion (98) as said catheter atherotome (10) is moved in the proximal direction in said artery.

25. The catheter atherotome (10) of claim 1 wherein said blades (34) define a cutting diameter (see Figure 3) when in said outwardly bowed arcuate configuration, and wherein said means (16, 18) for moving said inner member includes means (24) defining a scale (24) for indicating the position of said inner member (14) with respect to said outer sheath (12) and thus providing an indication of the size of said cutting diameter within said artery and for measuring said artery following removal of said plaque (98) therefrom.

26. The catheter atherotome (Figure 10) of claim 1 wherein at least one of said blades (34d) is free of a curved sharpened edge and located opposite a respective one of said blades (24c) defining a curved sharpened edge (44c), so as to contact an interior surface of an artery and support said curved sharpened edges (44c) with respect to said interior surface of said artery when said blades (44c) are in said radially outwardly bowed arcuate configuration.

27. The catheter atherotome (10) of claim 1, said elongate blades (34) defining a diameter of said catheter atherotome (10) and each said elongate blade (34) having a pitch with respect to said inner member (14), and said catheter atherotome further including means (16, 18) for controlling said pitch and said diameter while using said catheter atherotome (10).

## Patentansprüche

1. Ein Katheter zur Atherotomie (10) zur Verwendung bei chirurgischer Entfernung von Plaque von der Innenwand einer Arterie, umfassend:
(a) eine röhrenförmige äußere längliche Hülse (12), die jeweilige proximale und distale Enden (23 und 35) aufweist;
(b) ein röhrenförmiges längliches Innenstück (14), das innerhalb der äußeren Hülse angeordnet ist und jeweilige proximale und distale Enden (21, 37) aufweist, wobei besagtes distale Ende des besagten Innenstücks sich über besagtes distale Ende besagter äußeren Hülse hinaus erstreckt;
(c) eine Vielzahl von länglichen Klingen (34), von denen jede ein proximales Ende (40), das mit besagter äußeren Hülse (12) verbunden ist, und ein distales Ende (38), das mit besagtem Innenstück verbunden ist, aufweist;
(d) Mittel (16, 18) zum Bewegen besagten Innenstücks (14) bezüglich besagter äußeren Hülse (12), um besagtes distale Ende besagten Innenstücks zu besagtem distalen Ende von besagter äußeren Hülse zurückzuziehen und besagte Klingen in eine radial nach außen gebogene bogenförmige Konfiguration zu biegen; und
(e) wenigstens einige der besagten Klingen, die jeweilige gebogene geschärfte Schneiden (44) definieren, wobei wenigstens ein Teil einer jeden besagten gebogenen geschärften Schneide sich entlang besagtem Innenstück erstreckt und sich mehr zu besagtem proximalen Ende orientiert als zu besagtem distalen Ende des besagten Innenstücks, wenn besagte Klingen sich in besagter nach außen gebogener bogenförmiger Konfiguration befinden, um Plaque (98), die mit der inneren Wand einer Arterie verbunden ist, schneidend anzugreifen, während besagter Katheter zur Atherotomie (10) innerhalb besagter Arterie in proximaler Richtung bewegt wird, ohne besagte Innenwand besagter Arterie schneidend anzugreifen, während besagter Katheter zur Atherotomie (10) innerhalb besagter Arterie in distaler Richtung bewegt wird, wobei mindestens einige der besagten gebogenen geschärften Schneiden (44) jeweilige gegenüberliegende erste und zweite Enden (38, 40) aufweisen, wobei besagte ersten und zweiten Enden (38, 40) derselben durch einen kontrollierbar variablen Winkel über besagtem Innenstück (14) winkelig voneinander getrennt sind, wenn besagte Klingen (34) sich in besagter nach außen gebogener bogenförmigen Konfiguration befinden.

2. Der Katheter zur Atherotomie (10) nach Anspruch 1, wobei besagtes Innenstück (14) Lumenmittel (62) zur Aufnahme eines Führungsdrahtes definiert, der sich durch besagtes Innenstück (14) erstreckt, um besagten Katheter zur Atherotomie (10) innerhalb besagter Arterie zu führen.

3. Der Katheter zur Atherotomie (10) nach Anspruch 1, wobei besagtes Mittel (16, 18) zum Bewegen besagten Innenstücks (14) hebelbetriebenes Mittel (16, 18) zum Bewegen besagten Innenstückes (14) in Längsrichtung bezüglich besagter äußerer Hülse (12) einschließt.

4. Der Katheter zur Atherotomie (10) nach Anspruch 1, wobei besagtes Mittel (16, 18) zum Bewegen besagten Innenstückes (14) Mittel (20) einschließt, das mit besagtem Innenstück (14) verbunden ist, um besagtes Innenstück (14) bezüglich besagter äußeren Hülse (12) um eine begrenzte Winkelentfernung zu drehen, um besagten in kontrollierbarer Weise variablen Winkel um besagtes Innenstück (14) zu variieren und die Neigung besagter gebogener geschärfter Schneiden (44) bezüglich besagtem Innenstück (14) und somit bezüglich einer stenotischen Läsion (98), die mit besagtem Katheter zur Atherotomie (10) geschnitten werden soll, zu ändern.

5. Der Katheter zur Atherotomie (10) nach Anspruch 1, wobei jede der besagten länglichen Klingen (34) flexible Teile (46, 48) derselben einschließt, die keine Schneide derselben definieren.

6. Der Katheter zur Atherotomie (10) nach Anspruch 1, wobei diejenigen der besagten Klingen (34), die über in etwa die Hälfte des Umfangs der besagten äußeren Hülse (12) und des Innenstückes (14) angeordnet sind, besagte scharfe Schneiden (44) einschließen, wohingegen der Rest (46, 48) der besagten Klingen frei von besagten scharfen Schneiden sind.

7. Der Katheter zur Atherotomie (10) nach Anspruch 1, wobei jede der besagten Klingen (34), die eine gebogene geschärfte Schneide (44) definiert, ein Mittelstück (42), das besagte gebogene geschärfte Schneide (44) definiert, und ein Paar zu besagtem Mittelstück benachbarte Zwischenstücke (46, 48) umfaßt, wobei besagte Zwischenstücke (46, 48) wesentlich flexibler als besagtes Mittelstück (42) sind, daß besagtes Mittelstück (42) im wesentlichen seine ursprüngliche Form beibehält, während besagte Klingen (34) in besagte radial nach außen gebogene bogenförmige Konfiguration gebogen werden.

8. Der Katheter zur Atherotomie (10) nach Anspruch 1, wobei jede der besagten Klingen (34) ein Endstück (38, 40) aufweist, das sich jedem Ende derselben unmittelbar anschließt, und besagtes Innenstück (14) eine zentrale Längsachse definiert, der Katheter zur Atherotomie (10) Mittel (70, 72) umfaßt, um mindestens eines der besagten Enden einer jeden der besagten Klingen daran zu hindern, sich bezüglich besagter äußeren Hülse (12) und besagtem Innenstück (14) um eine sich parallel zu besagter zentralen Längsachse erstreckenden Achse zu drehen, während sie erlauben, daß sich ein jeweiliges der besagten Endstücke (38, 40) einer jeden der besagten Klingen (34) in einer radialen Ebene, die besagte zentrale Längsachse einschließt, zwischen einer Position, in der sich besagtes Endstück parallel zur besagten Längsachse erstreckt, und einer Position dreht, in der besagtes Endstück von besagter Längsachse in einem größeren spitzen Winkel wegweist und besagtes Mittelstück besagter Klinge von besagter Längsachse radial weiter weg versetzt ist.

9. Der Katheter zur Atherotomie (10) nach Anspruch 8, der jeweiliges Befestigungsmittel (Figuren 22, 23) umfaßt, das an mindestens besagter äußeren Hülse (12) und/oder besagtem Innenstück (14) befestigt ist, zum Befestigen besagter Klingen (34) an besagtem Innenstück (14) und besagter äußeren Hülse (12), wobei jedes besagtes Befestigungsmittel (Figuren 22, 23) einen Schwenkring (212) und ein jeweiliges Endstück (136) von einer jeden der besagten Klingen (34) umfaßt, das gebogen ist, um ein Nutmittel (220) zur Aufnahme eines jeweiligen Teils besagten Schwenkrings (212) zu definieren.

10. Der Katheter zur Atherotomie (10) nach Anspruch 1, wobei jede der besagten gebogenen geschärften Schneiden (44) von besagten Klingen (34) in einer Schneideposition angeordnet ist, die zu besagtem proximalen Ende (23) von besagter äußeren Hülse (12) exponiert ist und sich im allgemeinen in Umfangsrichtung um besagtes Innenstück (14) erstreckt, wenn besagtes distales Ende (37) von besagtem Innenstück (14) zu besagtem distalen Ende (35) von besagter äußeren Hülse (17) zurückgezogen wird und besagte Klingen (34) sich in der besagten nach außen gebogenen bogenförmigen Konfiguration befinden.

11. Der Katheter zur Atherotomie (10) nach Anspruch 1, wobei besagte Klingen (34) sich allgemein helikal erstrecken und besagte geschärften Schneiden (44) sich allgemein helikal erstrecken und zu besagtem proximalen Ende (23) von besagtem äußeren Hülsenstück (12) exponiert sind, wenn besagte Klingen (34) sich in besagter nach außen gebogener Konfiguration befinden.

12. Der Katheter zur Atherotomie (10) nach Anspruch 1, wobei besagtes Innenstück (14) röhrenförmig ist, weiterhin Kathermittel (30) mit einem Ballon an der Spitze umfaßt, das im sich durch besagtes Innenstück (14) Erstreckenden angeordnet ist, und eine Spitze (60) mit einem aufblasbaren Ballon aufweist, die distal jenseits besagtem distalen Ende des besagten Innenstückes (14) und Klingen (34) angeordnet ist, um Materialstücke, die von einem Athermom (98) während der Verwendung des besagten Katheters zur Atherotomie (10) freigeschnitten werden, zurückzuhalten und herauszuholen.

13. Der Katheter zur Atherotomie (10) nach Anspruch 1, wobei besagte Vielzahl von Klingen (34) einen Korb (32) definieren, wenn besagtes distale Ende (38) des besagten Innenstücks (14) zu besagtem distalen Ende (35) der besagten äußeren Hülse (12) zurückgezogen wird, besagter Korb (32) einen Äquatorteil definiert und besagte jeweiligen der besagten gebogenen geschärften Schneiden (44) der besagten Klingen (34) entlang besagtem Äquatorteil besagten Korbes (32) angeordnet sind und allgemein helikal um besagte äußere Hülse (14) des besagten Katheters zur Atherotomie angeordnet sind.

14. Der Katheter zur Atherotomie (10) nach Anspruch 1, wobei jede der besagten Klingen (34) ein Paar von gegenüberliegenden Zwischenstücken (46, 48) und ein Mittelstück (42) unfaßt, besagte Zwischenstücke federnd flexibel und verdrehbar sind und mindestens einige der besagten Mittelstücke jeweilige der besagten gebogenen geschärften Schneiden (44) umfassen, jede besagte gebogene geschärfte Schneide (44) normalerweise helikal um eine zentrale Längsachse des besagten Innenstückes (14) orientiert ist und einen Anstellwinkel definiert, besagter Anstellwinkel flach genug ist, so daß besagte gebogene geschärfte Schneide (44) gewöhnlicherweise die Oberfläche des Innengewebes einer gesunden Arterie nicht durch Schneiden angreifen wird, aber große genug ist, so daß besagte gebogene geschärfte Schneide (44) atherosklerotischen Plaque (98), die von den Innenoberflächen einer Arterie vorspringt, durch Schneiden angreifen wird, besagte Zwischenteile (46, 48) drehelastisch genug sind, um besagten Mittelstücken (42) besagter Klingen (34) zu erlauben, federnd bezüglich besagter Zwischenstücke (46, 48) in eine Position zu drehen, in der besagte scharfe Schneiden (44) einen Teil des besagten Plaquematerials (98) von besagten Innenoberflächen besagter Arterie angreifen und wegschneiden können.

15. Der Katheter zur Atherotomie nach Anspruch 1, weiterhin Mittel (262) zum Sammeln von abgeschabtem Material umfassend, das von Atherom (98) durch besagte Klingen (34) freigeschnitten wurde.

16. Der Katheter zur Atherotomie (10) nach Anspruch 1, wobei einige der Klingen (34) frei von scharfen Schneiden sind und bezüglich besagtem Innenstück (14) gegenüberliegend den besagten Klingen (34) angeordnet sind, die gebogene geschärfte Schneiden (44) definieren, so daß sie eine Innenoberfläche einer Arterie berühren und besagte Arterie dehnen, wenn besagte Klingen (34) sich in besagter radial nach außen gebogener bogenförmiger Konfiguration befinden.

17. Der Katheter zur Atherotomie (10) nach Anspruch 1, wobei mindestens eine der besagten Klingen (34) eine bandähnliche Struktur (42) aufweist, mit einem vorderen Rand (vorderer Teil von 44) und einem hinteren Rand (hinterer Teil von 44), besagter vorderer Rand umfaßt eine scharfe Schneide, besagtes proximales und distales Ende von besagter Klinge umfassen weiterhin jeweilige Endstücke (40, 50), und jedes besagte Endstück definiert jeweiliges Stegmittel zum Befestigen besagten Endstückes an die jeweilige besagte äußere Hülse (12) und das jeweilige besagte Innenstück (14).

18. Der Katheter zur Atherotomie (Figur 16) nach Anspruch 1, wobei mindestens eine der besagten Klingen (171) einen Körper (172) aus geformten Material aufweist und besagte scharfe Schneide (174') auf einem daran befestigten Einsatzteil (176) angeordnet ist.

19. Der Katheter zur Atherotomie (Figuren 30, 31a, 31b und 31c) nach Anspruch 1, wobei mindestens eine der besagten Klingen (282) aus Blech ist und ein Mittelstück (282) und ein Paar gegenüberliegender Endstücke aufweist, wobei besagte Endstücke bezüglich besagtem Mittelstück (282) winkelig versetzt sind.

20. Der Katheter zur Atherotomie (Figur 20) nach Anspruch 1, wobei mindestens eine der besagten Klingen (200) ein Körperteil (202) mit relativ flexibler Struktur und ein weniger flexibles, scharfschneidiges Einsatzteil (206) umfaßt, das starr mit besagtem Körperteil (202) verbunden ist und eine jeweilige der besagten scharfen Schneiden (208) definiert.

21. Der Katheter zur Atherotomie (Figur 14) nach Anspruch 1, wobei mindestens eine der besagten Klingen (124) ein Paar gegenüberliegender Endstücke (136) und ein Mittelstück (126), das sich dazwischen erstreckt, umfaßt, besagtes Mittelstück (126) eine geschärfte Schneide (142) umfaßt, die sich zwischen besagten Endstücken (136) erstreckt, besagte geschärfte Schneide (142) ein vorderes Teil (144) umfaßt, das im wesentlichen bezüglich besagter äußeren Hülse (12) radial ausgerichtet ist.

22. Der Katheter zur Atherotomie (260) nach Anspruch 1, wobei besagtes Innenstück (271) ein Lumen (62) definiert und besagtes zweites Ende (270) von besagtem Filter (262) mit einem distalen Ende besagten Innenstückes (14) verbunden ist.

23. Der Katheter zur Atherotomie (Figuren 20, 31a, 31b und 31c) nach Anspruch 1, der ein Spannglied (284) umfaßt, das sich parallel mit dem räumlich Entfernten von besagter scharfer Schneide (288) einer der besagten Klingen (282) erstreckt, die vollständige umfangsmäßige Erfassung der besagten Arterie vorsehen, um durch eine stenotische Läsion (98) zu schneiden, während besagter Katheter zur Atherotomie (10) in besagter Arterie in proximaler Richtung bewegt wird.

24. Der Katheter zur Atherotomie (10) nach Anspruch 1, wobei alle besagten Klingen (34) jeweilige gebogene geschärfte Schneiden (44) umfassen, die einander über besagtem Innenstück (14) überlappen, wenn sich besagte Klingen (34) in der nach außen gebogenen bogenförmigen Konfiguration befinden und besagtes Innenstück (14) sich in einer vorab bestimmten winkeligen Position bezüglich besagter äußeren Hülse (12) befindet, der vollständige umfangsmäßige Erfassung der besagten Arterie vorsieht, um durch eine stenotische Läsion (98) zu schneiden, während besagter Katheter zur Atherotomie (10) in besagter Arterie in proximaler Richtung bewegt wird.

25. Der Katheter zur Atherotomie (10) nach Anspruch 1, wobei besagte Klingen (34) einen Schnittdurchmesser (siehe Figur 3) definieren, wenn sie sich in besagter nach außen gebogener bogenförmiger Konfiguration befinden, und wobei besagtes Mittel (16, 18) zum Bewegen besagten Innenstückes Mittel (24) umfaßt, das eine Skala (24) zum Anzeigen der Position des besagten Innenstückes (14) bezüglich besagter äußeren Hülse (12) definiert und damit eine Anzeige der Größe besagten Schnittdurchmessers innerhalb besagter Arterie und zum Messen besagter Arterie nach Entfernung der besagten Plaque (98) davon vorsieht.

26. Der Katheter zur Atherotomie (Figur 10) nach Anspruch 1, wobei mindestens eine der besagten Klingen (34d) frei von einer gebogenen geschärften Schneide ist und gegenüber einer jeweiligen der besagten Klingen (24c) angeordnet ist, die eine gebogene geschärfte Schneide (44c) definieren, um eine innere Oberfläche einer Arterie zu berühren und besagte gebogene geschärfte Schneide (44c) bezüglich besagter inneren Oberfläche der besagten Arterie zu unterstützen, wenn besagte Klingen (44c) sich in besagter radial nach außen gebogener bogenförmigen Konfiguration befinden.

27. Der Katheter zur Atherotomie (10) nach Anspruch 1, wobei besagte längliche Klingen (34) einen Durchmesser des besagten Katheters zur Atherotomie (10) definieren und jede besagt längliche Klinge (34) eine Ganghöhe bezüglich des Innenstückes (14) aufweist, und besagter Katheter zur Atherotomie weiterhin Mittel (16, 18) umfaßt, um besagte Ganghöhe und besagten Durchmesser zu kontrollieren, während besagter Katheter zur Atherotomie (10) verwendet wird.

## Revendications

1. Un athérotome à cathéter (10) destiné au retrait chirurgical de plaque de la paroi intérieure d'une artère comprenant :
(a) un fourreau externe tubulaire allongé (12) ayant des extrémités proximale et distale respectives (23 et 35) ;
(b) un élément interne tubulaire allongé (14) disposé dans ledit fourreau externe et ayant des extrémités proximale et distale respectives (21, 37), ladite extrémité distale dudit élément interne s'étendant au delà de ladite extrémité distale dudit fourreau externe ;
(c) une pluralité de lames allongées (34) ayant chacune une extrémité proximale (40) interconnectée audit fourreau externe (12) et une extrémité distale (38) interconnectée audit élément interne ;
(d) un moyen (16, 18) pour déplacer ledit élément interne (14) par rapport audit fourreau externe (12) de manière à rétracter ladite extrémité distale dudit élément interne vers ladite extrémité distale dudit fourreau externe et fléchir lesdites lames en une configuration arquée inclinée vers l'extérieur radialement ; et
(e) au moins certaines desdites lames définissant des bords tranchants courbes respectifs (44), au moins une portion de chaque dit bord tranchant courbe s'étendant le long dudit élément interne et étant orientée plus vers ladite extrémité proximale que vers ladite extrémité distale dudit élément interne lorsque lesdites lames sont dans ladite configuration arquée inclinée vers l'extérieur, de manière à s'engager de manière coupante dans la plaque (98) associée à une paroi interne d'une artère lorsque ledit athérotome à cathéter (10) est déplacé dans ladite artère suivant une direction proximale, sans s'engager de manière coupante dans ladite paroi interne de ladite artère lorsque ledit athérotome à cathéter (10) est déplacé dans ladite artère suivant une direction distale, au moins certains desdits bords tranchants courbes (44) ayant des première et seconde extrémités opposées respectives (38, 40), lesdites première et seconde extrémités (38, 40) de ceux-ci étant séparées de manière angulaire de chaque autre par un angle variable contrôlable par rapport audit élément interne (14) lorsque lesdites lames (34) sont dans ladite configuration arquée inclinée vers l'extérieur.

2. L'athérotome à cathéter (10) de la revendication 1 dans lequel ledit élément interne (14) définit un moyen de passage (62) pour recevoir un fil de guidage s'étendant à travers ledit élément interne (14) pour guider ledit athérotome à cathéter (10) dans ladite artère.

3. L'athérotome à cathéter (10) de la revendication 1 dans lequel ledit moyen (16, 18) pour déplacer ledit élément interne (14) inclut un moyen fonctionnant par levier (16, 18) pour déplacer ledit élément interne (14) longitudinalement par rapport audit fourreau externe (12).

4. L'athérotome à cathéter (10) de la revendication 1 dans lequel ledit moyen (16, 18) pour déplacer ledit élément interne (14) inclut un moyen (20) associé audit élément interne (14) pour tourner ledit élément interne (14) par rapport audit fourreau externe (12) sur une distance angulaire limitée, de manière à faire varier ledit angle variable contrôlable autour dudit élément interne (14) et modifier l'inclinaison desdits bords tranchants courbes (44) par rapport audit élément interne (14) et ainsi par rapport à une lésion sténotique (98) à couper par ledit athérotome à cathéter (10).

5. L'athérotome à cathéter (10) de la revendication 1 dans lequel chacune desdites lames allongées (34) inclut des portions flexibles (46, 48) de celle-ci qui ne définissent pas un bord de celle-ci.

6. L'athérotome à cathéter (10) de la revendication 1 dans lequel les unes desdites lames (34) disposées approximativement sur une moitié de la périphérie desdits fourreau extérieur (12) et élément interne (14) incluent lesdits bords tranchants (44), tandis que le reste (46, 48) desdites lames est exempt desdits bords tranchants.

7. L'athérotome à cathéter (10) de la revendication 1 dans lequel chacune desdites lames (34) définissant un bord tranchant courbe (44) inclut une portion médiane (42) définissant ledit bord tranchant courbe (44) et une paire de portions intermédiaires (46, 48) adjacentes à ladite portion médiane, lesdites portions intermédiaires (46, 48) étant suffisamment plus flexibles que ladite portion médiane (42), laquelle dite portion médiane (42) demeure sensiblement à sa forme d'origine pendant le fléchissement desdites lames (34) en ladite configuration arquée inclinée vers l'extérieur radialement.

8. L'athérotome à cathéter (10) de la revendication 1 dans lequel chacune desdites lames (34) a une portion terminale (38, 40) proche de chaque extrémité de celle-ci et ledit élément interne (14) définit un axe longitudinal central, l'athérotome à cathéter (10) incluant un moyen (70, 72) pour empêcher au moins l'une desdites extrémités de chacune desdites lames de tourner par rapport audit fourreau externe (12) et ledit élément interne (14) autour d'un axe s'étendant parallèlement audit axe longitudinal central, tout en permettant à l'une respective desdites portions terminales (38, 40) de chacune desdites lames (34) de tourner dans un plan radial incluant ledit axe longitudinal central, entre une position à laquelle ladite portion terminale s'étend parallèlement audit axe longitudinal et une position à laquelle ladite portion terminale s'écarte dudit axe longitudinal avec un angle aigu plus grand et ladite portion médiane de ladite lame est déplacée radialement plus loin dudit axe longitudinal.

9. L'athérotome à cathéter (10) de la revendication 8 incluant un moyen de montage respectif (Figures 22, 23) lié au moins à l'un dudit fourreau externe (12) et dudit élément interne (14) pour une liaison desdites lames (34) audit élément interne (14) et audit fourreau externe (12), chaque moyen de montage (figures 22, 23) incluant une bague de pivot (212), et une portion terminale respective (136) de chacune desdites lames (34) étant coudée pour définir un moyen de canal (220) pour recevoir une portion respective de ladite bague de pivot (212).

10. L'athérotome à cathéter (10) de la revendication 1 dans lequel chacun desdits bords tranchants courbes (44) desdites lames (34) est localisé à une position de coupe exposée vers ladite extrémité proximale (23) dudit fourreau externe (12) et s'étendant généralement à la périphérie autour dudit élément interne (14) lorsque ladite extrémité distale (37) dudit élément interne (14) est rétractée vers ladite extrémité distale (35) dudit fourreau externe (12) et lesdites lames (34) sont dans ladite configuration arquée inclinée vers l'extérieur.

11. L'athérotome à cathéter (10) de la revendication 1 dans lequel lesdites lames (34) s'étendent en général hélicoïdalement et lesdits bords tranchants (44) s'étendent en général hélicoïdalement et sont exposés vers ladite extrémité proximale (23) dudit élément de fourreau externe (12) lorsque lesdites lames (34) sont dans ladite configuration inclinée vers l'extérieur.

12. L'athérotome à cathéter (10) de la revendication 1 dans lequel ledit élément interne (14) est tubulaire, incluant en outre un moyen de cathéter terminé en ballon (30), disposé dans le s'étendant à travers ledit élément interne (14) et ayant un bout en ballon gonflable (60) localisé de manière distale au-delà de ladite extrémité distale desdits élément interne (14) et lames (34), pour retenir et récupérer des morceaux de matière coupés sans un athérome (98) pendant l'usage dudit athérotome à cathéter (10).

13. L'athérotome à cathéter (10) de la revendication 1, ladite pluralité de lames (34) définissant un panier (32) lorsque ladite extrémité distale (38) dudit élément interne (14) est rétractée vers ladite extrémité distale (35) dudit fourreau externe (12), ledit panier (32) définissant une portion équatoriale, et lesdits uns respectifs desdits bords tranchants courbes (44) desdites lames (34) étant localisés le long de ladite portion équatoriale dudit panier (32) et étant orientés en général hélicoïdalement autour dudit fourreau externe (14) dudit athérotome à cathéter.

14. L'athérotome à cathéter (10) de la revendication 1 dans lequel chacune desdites lames (34) inclut une paire de portions intermédiaires opposées (46, 48) et une portion médiane (42), lesdites portions intermédiaires étant flexibles de manière résiliente et pouvant être vrillées, et au moins certaines desdites portions médianes incluant les uns respectifs desdits bords tranchants courbes (44), chaque dit bord tranchant courbe (44) étant normalement orienté hélicoïdalement autour d'un axe longitudinal central dudit élément interne (14), et définissant un angle d'incidence, ledit angle d'incidence étant assez peu prononcé afin que ledit bord tranchant courbe (44) normalement ne s'engage pas de manière coupante dans la surface du tissu interne d'une artère saine mais étant assez grand afin que ledit bord tranchant courbe (44) s'engage de manière coupante dans la plaque athérosclérotique (98) saillant des surfaces intérieures d'une artère, lesdites portions intermédiaires (46, 48) étant assez élastiques en torsion pour permettre auxdites portions médianes (42) desdites lames (34) de tourner de manière résiliente par rapport auxdites portions intermédiaires (46, 48) à une positon à laquelle lesdits bords tranchants (44) peuvent s'engager dans et rogner une portion de ladite matière de plaque (98) depuis lesdites surfaces intérieures de ladite artère.

15. L'athérotome à cathéter (10) de la revendication 1, incluant en outre un moyen (262) pour collecter des rognures de matière coupées sans athérome (98) par lesdites lames (34).

16. L'athérotome à cathéter (10) de la revendication 1 dans lequel certaines desdites lames (34) sont exemptes de bords tranchants et sont localisées à l'opposé desdites lames (34) définissant des bords tranchants courbes (44), par rapport audit élément interne (14), de manière à mettre en contact une surface intérieure d'une artère et à distendre ladite artère lorsque lesdites lames (34) sont dans ladite configuration arquée inclinée vers l'extérieur radialement.

17. L'athérotome à cathéter (10) de la revendication 1 dans lequel au moins l'une desdites lames (34) est d'une forme analogue à un ruban (42), ayant une bordure d'attaque (partie avant de 44) et une bordure arrière (partie arrière de 44), la bordure d'attaque incluant un bord tranchant, lesdites extrémités proximale et distale de ladite lame incluant en outre des portions terminales respectives (40, 50), et chaque dite portion terminale définissant un moyen de pince respectif (40, 50) pour lier ladite portion terminale à l'un respectif dudit fourreau externe (12) et dudit membre interne (14).

18. L'athérotome à cathéter (Figure 16) de la revendication 1 dans lequel au moins l'une desdites lames (171) a un corps (172) en matière moulée et ledit bord tranchant (174') est localisé sur un insert (176) lié à celui-ci.

19. L'athérotome à cathéter (Figures 30, 31a, 31b, et 31c) de la revendication 1 dans lequel au moins l'une desdites lames (282) est en feuille métallique et a une portion médiane (282) et une paire de portions terminales opposées, lesdites portions terminales étant décalées angulairement par rapport à ladite portion médiane (282).

20. L'athérotome à cathéter (Figure 20) de la revendication 1 dans lequel au moins l'une desdites lames (200) inclut une portion de corps (202) de structure relativement flexible et une portion d'insert à bord tranchant, moins flexible (206), liée rigidement à ladite portion de corps (202) et définissant l'un respectif desdits bords tranchants (208).

21. L'athérotome à cathéter (Figure 14) de la revendication 1 dans lequel au moins l'une desdites lames (124) inclut une paire de portion terminale opposée (136) et une portion médiane (126) s'étendant entre elles, ladite portion médiane (126) incluant un bord tranchant (142) s'étendant entre lesdites portions terminales (136), ledit bord tranchant (142) incluant une portion d'attache (144) dirigée sensiblement radialement par rapport audit fourreau externe (12).

22. L'athérotome à cathéter (260) de la revendication 1 dans lequel ledit élément interne (271) définit un passage (62) et ladite seconde extrémité (270) dudit filtre (262) est liée à une extrémité distale dudit élément interne (14).

23. L'athérotome à cathéter (Figures 20, 31a, 31b et 31c) de la revendication 1 incluant un élément raidisseur (284) s'étendant parallèlement audit, mais espacé dudit bord tranchant (288) de l'une desdites lames (282), fournissant une couverture périphérique complète de ladite artère de manière à couper à travers une lésion sténotique (98) lorsque ledit athérotome à cathéter (10) est déplacé suivant la direction proximale dans ladite artère.

24. L'athérotome à cathéter (10) de la revendication 1 dans lequel toutes lesdites lames (34) incluent des bords tranchants courbes respectifs (44) se chevauchant autour dudit élément interne (14) lorsque lesdites lames (34) sont dans ladite configuration arquée inclinée vers l'extérieur et ledit élément interne (14) est à une position angulaire prédéterminée par rapport audit fourreau externe (12), fournissant une couverture périphérique complète de ladite artère de manière à couper à travers une lésion sténotique (98) lorsque ledit athérotome à cathéter (10) est déplacé dans la direction proximale dans ladite artère.

25. L'athérotome à cathéter (10) de la revendication 1 dans lequel lesdites lames (34) définissent un diamètre de coupe (voir figure 3) lors de ladite configuration arquée inclinée vers l'extérieur, et dans lequel ledit moyen (16, 18) pour déplacer ledit élément interne inclut un moyen (24) définissant une échelle (24) pour indiquer la position dudit élément interne (14) par rapport audit fourreau externe (12) et ainsi fournir une indication de la dimension dudit diamètre de coupe dans ladite artère et pour mesurer ladite artère faisant suite au retrait de ladite plaque (98) de celle-ci.

26. L'athérotome à cathéter (Figure 10) de la revendication 1 dans lequel au moins l'une desdites lames (34d) est exempte de bord tranchant courbe et localisée à l'opposé d'une respective desdites lames (24c) définissant un bord tranchant courbe, de manière à mettre en contact une surface intérieure d'une artère et supporter lesdits bords tranchants courbes (44c) par rapport à ladite surface intérieure de ladite artère lorsque lesdites lames (44c) sont dans ladite configuration arquée inclinée vers l'extérieur radialement.

27. L'athérotome à cathéter (10) de la revendication 1, lesdits lesdites lames allongées (34) définissant un diamètre dudit athérotome à cathéter (10) et chaque dite lame allongée (34) ayant un écartement par rapport audit élément interne (14), et ledit athérotome à cathéter incluant en outre un moyen (16, 18) pour contrôler ledit écartement et ledit diamètre lors de l'usage dudit athérotome à cathéter (10).
